**Europäisches Patentamt**

(19) **European Patent Office**

**Office européen des brevets**

(11) Numéro de publication: **0 169 828**

**B1**

(12) **FASCICULE DE BREVET EUROPÉEN**

(45) Date de publication du fascicule du brevet:
**12.10.88**

(21) Numéro de dépôt: **85870101.4**

(22) Date de dépôt: **19.07.85**

(51) Int. Cl.⁴: **C 07 C 7/148**

(54) Procédé pour enlever le sulfure de carbonyle des charges d'hydrocarbures liquides oléfiniques.

(30) Priorité: **25.07.84 LU 85478**

(43) Date de publication de la demande:
**29.01.86 Bulletin 86/5**

(45) Mention de la délivrance du brevet:
**12.10.88 Bulletin 88/41**

(84) Etats contractants désignés:
**AT BE DE FR GB IT NL**

(56) Documents cité:
**EP-A-0 016 284**
**FR-A-1 483 583**

(73) Titulaire: **LABOFINA S.A., 52, rue de l'Industrie, B-1040 Bruxelles (BE)**

(72) Inventeur: **Debras, Guy L.G., 176, Avenue Joseph Abras, B-5001 Belgrade (BE)**
Inventeur: **De Clippeleir, Georges E.M.J., 5, Petrus Huysegomstraat, B-1600 Sint Pieters Leeuw (BE)**
Inventeur: **Cahen, Raymond M., 21, Avenue du Commandant Lothaire, B-1040 Bruxelles (BE)**

(74) Mandataire: **Detrait, Jean- Claude, LABOFINA S.A. Zoning Industriel, B-6520 Feluy (BE)**

LIBER, STOCKHOLM 1988

## Description

La présente invention se rapporte à un procédé pour enlever le soufre présent sous forme d'oxysulfure de carbone ou sulfure de carbonyle, hors d'hydrocarbures liquides, en particulier les charges d'hydrocarbures oléfiniques contenant notamment du propylène.

Dans les raffineries, le traitement des hydrocarbures liquides pour l'enlèvement ou pour la transformation des impuretés nécessite des procédés complexes et coûteux. Les divers composés sulfurés représentent les impuretés habituelles que l'on cherche à éliminer. Ceux-ci sont l'acide sulfhydrique, les mercaptans et en particulier le sulfure de carbonyle.

La pratique qui prévaut dans l'industrie consiste à réduire la teneur en soufre en traitant les hydrocarbures à l'état gazeux. Une pratique largement acceptée consiste à enlever les sulfures hors de gaz combustibles pris à l'état gazeux avec la diéthanolamine, la diisopropanolamine, la monoéthanolamine, et du tétraéthylène glycol. On sait également que ces solvants peuvent être utilisés pour traiter les hydrocarbures à l'état liquide.

Cependant, lorsque ces solvants sont utilisés pour extraire les impuretés hors d'hydrocarbures à l'état liquide, ils ne permettent pas de réduire la teneur en sulfure de carbonyle, ci-après dénommé COS pour la facilité, à moins de 5 ppm.

Or, il est connu également que les nouveaux procédés de polymérisation de propylène font appel à des catalyseurs de plus en plus performants. Ces derniers, en contre-partie, sont extrêmement sensibles à toutes impuretés polaires comme par exemple le COS, dont le moment dipolaire est de 0,736 Debye. Lorsque l'on utilise ces procédés de polymérisation, il est donc important de purifier la charge de sorte que la teneur résiduelle en impuretés soit extrêmement faible.

Or, avec les procédés classiques qui traitent des charges qui peuvent contenir au départ de 30 à 70 ppm de COS, on parvient à atteindre une teneur résiduelle en COS après purification de l'ordre de 10 à 20 ppm.

On a déjà proposé de traiter des charges d'hydrocarbures liquides, contenant notamment du propylène, avec du 2-(2-aminoéthoxy) éthanol, connu sous le nom de diglycolamine, pour enlever le COS; cependant, la teneur résiduelle obtenue, bien que faible puisque de l'ordre du ppm, n'est pas encore suffisante pour satisfaire les conditions des polymérisations à l'aide des catalyseurs de type Ziegler très actifs.

A côté de la technique de traitement fa..ant appel à des contacts liquide-liquide, on a déjà proposé également des traitements par contacts liquide-solide.

Ce type de traitement a l'avantage de limiter les risques de contamination du propylène qui doit être polymérisé ultérieurement, contamination qui entraînerait la nécessité d'un second absorbeur.

Ainsi, on a déjà proposé (FR-A-1 483 583) d'utiliser un matériau solide constitué d'un support inerte poreux présentant une grande surface spécifique tel que le silicagel, pierre ponce ou silicate de Mg et des oxydes de Cd, Zn, Ni et Co sur lequel on fait passer la charge liquéfiee conduisant à une teneur résiduelle en COS de la charge inférieure à 1 ppm.

On a également proposé d'utiliser des absorbants composés d'oxyde de fer, d'oxyde de cuivre ou d'oxyde de cuivre et chrome sur un support à haute surface spécifique comme le charbon actif ou de l'alumine, de manière à réduire la teneur en COS des hydrocarbures liquides depuis 50-60 ppm dans la charge initiale jusqu'à environ 0,5 ppm.

Cependant, bien que cette teneur soit déjà très faible, cela n'est pas encore suffisant pour pouvoir envoyer le propylène ainsi purifié à l'unité de polymérisation utilisant les catalyseurs Ziegler très actifs.

On a aussi proposé de traiter ces charges en les faisant passer sur des résines basiques échangeuses d'ions, à la température ambiante. Cependant, la teneur résiduelle en COS obtenue est également de l'ordre du ppm, ce qui est trop élevé pour effectuer la polymérisation en présence de catalyseurs de polymérisation de la dernière génération.

On a également proposé d'utiliser de l'oxyde de zinc déposé sur un support d'alumine, cependant ce type de catalyseur n'est pas suffisamment actif pour pouvoir enlever le COS jusqu'à obtenir des teneurs résiduelles inférieures à 30 ppb.

On a également développé des procédés pour enlever le COS hors de gaz de synthèse, en présence d'oxyde de zinc comme catalyseur. Cependant, les conditions de traitement et les résultats obtenus en phase gazeuse sont loin d'être semblables à ceux obtenus en phase liquide; en outre, la nature de la charge à purifier joue un rôle important.

Il existe dès lors un besoin pour un procédé qui permette de désulfurer et en particulier qui permette d'enlever le COS des charges d'hydrocarbures liquides oléfiniques contenant notamment du propylène et d'autres oléfines, jusqu'à ce que la teneur résiduelle en COS ne dépasse pas 30 ppb, de manière à ce que les catalyseurs de polymérisation du propylène de la nouvelle génération ne soient pas trop rapidement empoisonnés.

La présente invention a pour but un procédé de purification des charges d'hydrocarbures liquides oléfiniques qui permette de satisfaire les critères énoncés ci-dessus.

La présente invention a pour objet un procédé de purification qui permette d'enlever le COS des charges d'hydrocarbures liquides oléfiniques contenant notamment du propylène, jusqu'à obtenir une teneur résiduelle en COS inférieure à 30 ppb.

Le procédé de purification de la présente invention pour enlever le COS des charges d'hydrocarbures liquides oléfiniques comprenant notamment du propylène et contenant de 1 à 70 ppm de COS, est caractérisé en ce que l'on fait passer la charge d'hydrocarbures liquides, à une vitesse spatiale horaire de liquide (LHSV) comprise entre 0,2 et 15, sur un matériau absorbant à base d'oxyde de zinc et d'un promoteur choisi parmi l'alumine, les silico-alumines, l'oxyde de calcium ou leurs mélanges, la quantité de promoteur ne dépassant pas 15 % en poids du catalyseur, la surface spécifique du matériau absorbant étant comprise entre 20 et 100 m²/g.

On a trouvé, de manière inattendue, qu'en faisant passer avec une LHSV de 0,2 à 15 une charge d'hydrocarbures oléfiniques liquides, en l'occurence une charge de propylène destiné à la polymérisation, sur un matériau absorbant constitué de 85 à 95 % en poids d'oxyde de zinc, 3 à 10 % en poids d'$Al_2O_3$ et de 0 à 5 % en poids d'oxyde de calcium, on obtient une charge purifiée répondant aux conditions de pureté exigées par une polymérisation en présence de catalyseur Ziegler de la dernière génération, c'est-à-dire notamment une teneur en COS ne dépassant pas environ 30 ppb.

La surface spécifique du matériau absorbant est comprise entre 20 et 100 m²/g. et de préférence entre 30 et 80 m²/g.

On peut évidemment utiliser des matériaux absorbants ayant une plus grande surface spécifique, mais l'avantage qui en résulte n'est pas très significatif. D'autre part, des matériaux ayant une surface spécifique inférieure à 20 m²/g. n'ont pas la capacité d'absorption suffisante et leur activité catalytique est beaucoup plus faible.

De plus, il est avantageux d'utiliser le matériau absorbant sous forme finement divisée. Généralement, la dimension des particules de ce matériau ne dépassera pas 2 mm et sera comprise le plus souvent entre 0,5 et 1,5 mm.

Le fait est d'autant plus inattendu que lorsque l'on traite des charges similaires avec des catalyseurs connus à base de ZnO, on est loin d'obtenir la pureté exigée pour le COS.

D'autre part, si l'on se réfère au traitement de gaz de synthèse en phase gazeuse, la teneur en eau de la charge permet déjà une hydrolyse du COS alors que c'est loin d'être le cas lors du traitement des charges de propylène qui sont et qui doivent être exemptes d'eau.

Généralement, on traite des charges d'hydrocarbures liquides contenant plus de 75 % de propylène, plus particulièrement des charges contenant jusqu'à 85 et 99 % de propylène, la teneur en COS étant généralement de l'ordre de 1 à 10 ppm. Lorsque l'on doit traiter des charges ayant une teneur plus élevée en COS, c'est-à-dire jusqu'à 500 ppm, on leur fait d'abord subir un traitement avec un solvant aminé, comme la monoéthanolamine, de manière à réduire le taux de COS à une valeur convenable, soit moins de 70 ppm.

Selon un mode d'exécution du procédé de la présente invention, on fait passer la charge d'hydrocarbures liquides contenant du propylène sur le matériau absorbant de la présente invention, à une température comprise généralement entre 0°C et 90°C et à une pression suffisante pour maintenir le milieu en phase liquide.

La vitesse spatiale horaire de liquide ou LHSV, à laquelle on fait passer la charge, est généralement comprise entre 0,1 et 20, et de préférence comprise entre 0,2 et 15.

On a remarqué que la quantité de promoteur est très critique. De fait, pour obtenir des teneurs résiduelles en COS aussi faibles que 30 ppb, il est necessaire d'ajouter une certaine quantité de promoteur à l'oxyde de zinc, cette quantité ne dépassant pas 15 % en poids.

Selon un mode préféré du procédé de l'invention, on utilise au moins de 3 à 10 % en poids d'alumine, comme promoteur, l'oxyde de calcium pouvant ne pas être présent. Cependant, on préfère utiliser un catalyseur qui comprend de l'oxyde de zinc et de 3 à 10 % d'alumine ou de silicoalumine et de 0,5 à 5 % d'oxyde de calcium. Lorsque l'on utilise des quantités plus élevées de ces promoteurs, on n'enlève pas suffisamment de COS pour respecter les conditions de pureté qui sont imposées aux charges de propylène destinées à la polymérisation.

Les exemples suivants sont donnés afin de mieux illustrer le procédé de la présente invention.

## Exemple 1

On a fait passer une charge d'hydrocarbures liquides, contenant 99 % de propylène et ayant une teneur résiduelle en COS de 2,7 ppm de COS, sur un matériau absorbant constitué par 92 % d'oxyde de Zinc, 5 % d'$Al_2O_3$ et 3 % de CaO, qui a été finement divisé de manière à avoir des particules dont la dimension moyenne est d'environ 1 mm.

La surface spécifique de ce matériau était de 44 m²/g.

On a ainsi fait passer la charge mentionnée ci-dessus sur le matériau absorbant, à température ambiante et à une pression ajustée pour maintenir la charge en phase liquide, et à une LHSV de 5.

On a pris un échantillon de la charge purifiée et on a déterminé la teneur en COS. Celle-ci était de 25 ppb.

## Exemple 2

On a fait passer une charge d'hydrocarbures liquides contenant 99 % de propylène et ayant une teneur résiduelle en COS de 1,5 ppm, sur un matériau absorbant utilisé à l'exemple 1.

La surface spécifique de ce matériau était de 44 m²/g.

On a ainsi fait passer la charge sur le matériau absorbant à température de 5°C, à une pression ajustée pour maintenir la charge en phase liquide à cette température et à une LHSV de 5.

On a pris un échantillon de la charge purifiée et on a déterminé la teneur en COS. Celle-ci était de 18 ppb.

A titre de comparaison, on a fait passer la même charge sur un matériau absorbant de 100 % en poids d'oxyde de zinc. Ce matériau avait une surface spécifique de 10 m²/g.

On a réalisé l'opération à température de 5°C et à une pression suffisante pour maintenir la charge en phase liquide.

On a fait passer cette charge sur l'absorbant à une LHSV de 5.

On a pris un échantillon de la charge purifiée et on a déterminé sa teneur en COS. Celle-ci était de 1,2 ppm.

Cet exemple comparatif montre l'importance de la présence du promoteur dans le catalyseur.

**Exemple 3**

On fait passer une charge d'hydrocarbures liquides, contenant 99 % de propylène et ayant une teneur résiduelle en COS de 1,5 ppm de COS, sur un matériau absorbant constitué par 89 % de ZnO, 7 % de $Al_2O_3$ et 4 % de CaO, qui a été finement divisé de manière à avoir des particules dont la dimension moyenne est inférieure à 2 mm, avec une forte proportion entre 1 et 1,5 mm.

La surface spécifique de ce matériau était de 30 m²/g.

On a ainsi fait passer la charge mentionnée ci-dessus par le matériau absorbant, à température de 5°C et à une pression ajustée pour maintenir la charge en phase liquide, et à une LHSV de 5.

On a pris un échantillon de la charge purifiée et on a déterminé la teneur en COS. Celle-ci était de 12 ppb.

On a fait passer la même charge sur le même matériau absorbant aux mêmes température et pression, mais à une LHSV de 17. Dans ces conditions, la teneur résiduelle en COS dans la charge purifiée était de 45 ppb.

A titre de comparaison, on a pris 45,6 g d'une alumine commerciale, sous forme de cylindre de 5 mm de diamètre, dont la surface spécifique est de 250 m²/g. On l'a calcinée à 500°C et on l'a imprégnée avec une solution de $Zn(NO_3)_2.4H_2O$

Le catalyseur final contenait 50,4 % en poids de ZnO, et la surface spécifique finale du matériau était de 153 m²/g.

On a traité la même charge que celle décrite ci-dessus sur le catalyseur, à une température de 5°C et à une pression suffisante pour maintenir la charge en phase liquide et à une LHSV de 5.

Dans ces conditions la teneur résiduelle en COS dans la charge purifiée était de 345 ppb, ce qui est inacceptable.

**Revendications**

1. Procédé de purification de charges d'hydrocarbures liquides oléfiniques comprenant notamment du propylène et contenant de 1 à 70 ppm de COS, pour enlever le sulfure de carbonyle, caractérisé en ce que l'on fait passer la charge d'hydrocarbures liquides à une vitesse spatiale horaire de liquide comprise entre 0,2 et 15 sur un matériau absorbant à base d'oxyde de zinc et d'un promoteur choisi parmi l'alumine, les silico-alumines, l'oxyde de calcium ou leurs mélanges, la quantité de promoteur ne dépassant pas 15 % en poids du matériau absorbant, la surface spécifique du matériau absorbant étant comprise entre 20 et 100 m²/g.

2. Procédé de purification selon la revendication 1, caractérisé en ce que l'on fait passer la charge d'hydrocarbures liquides oléfiniques sur un matériau absorbant constitué de 85 à 95 % en poids d'oxyde de zinc, de 3 à 10 % en poids d'alumine et de 0 à 5 % en poids d'oxyde de calcium.

3. Procédé selon l'une quelconque des revendications 1 et 2, caractérisé en ce que l'on fait passer la charge d'hydrocarbures liquides oléfiniques sur un matériau absorbant finement divisé, dont les dimensions de particules ne dépassent pas 2 mm et sont de préférence comprises entre 0,5 et 1,5 mm.

4. Procédé selon l'une quelconque des revendications 1 à 3, caractérisé en ce que la surface spécifique du matériau absorbant est comprise entre 30 et 80 m²/g.

5. Procédé selon l'une quelconque des revendications 1 à 4, caractérisé en ce que l'on fait passer la charge d'hydrocarbures liquides oléfiniques sur le matériau absorbant à une température comprise entre environ 0°C et environ 90°C et à une pression suffisante pour maintenir la charge en phase liquide.

6. Procédé selon l'une quelconque des revendications 1 à 5, caractérisé en ce que l'on fait passer une charge d'hydrocarbures liquides oléfiniques contenant plus de 75 % de propylène.

7. Procédé selon l'une quelconque des revendications 1 à 6, caractérisé en ce que l'on fait passer une charge d'hydrocarbures oléfiniques contenant plus de 95 % de propylène.

**Patentansprüche**

1. Verfahren zum Reinigen von Chargen von olefinischen flüssigen Kohlenwasserstoffen, die insbesondere Propylen umfassen und 1 bis 70 ppm COS enthalten, um Schwefelsulfid zu entfernen, dadurch gekennzeichnet, daß die Charge flüssiger Kohlenwasserstoffe mit einer stündlichen Flüssigkeitsraumgeschwindigkeit zwischen 0,2 und 15 über ein Absorptionsmittel auf der Basis von Zinkoxid und einem Promotor, der unter Aluminiumoxid, Silicoaluminiumoxiden, Calciumoxid und Gemischen derselben ausgewählt worden ist, hinweggeleitet wird, wobei die Promotormenge 15 Gew.-% des Absorptionsmittels nicht übersteigt und wobei die wirksame Oberfläche des Absorptionsmittel zwischen 20 und 100 m²/g liegt.

2. Reinigungsverfahren nach Anspruch 1, dadurch gekennzeichnet, daß die Charge von olefinischen flüssigen Kohlenwasserstoffen über ein Absorptionsmittel geleitet wird, das aus 85 bis 95 Gew.-% Zinkoxid, 3 bis 10 Gew.-% Aluminiumoxid und 0 bis 5 Gew.-% Calciumoxid besteht.

3. Verfahren nach einem der Ansprüche 1 und 2, dadurch gekennzeichnet, daß die Charge von olefinischen flüssigen Kohlenwasserstoffen über ein fein verteiltes Absorptionsmittel geleitet wird, dessen Teilchenabmessungen 2 mm nicht überschreiten und vorzugsweise zwischen 0,5 und 1,5 mm liegen.

4. Verfahren nach einem der Ansprüche 1 bis 3, dadurch gekennzeichnet, daß die wirksame Oberfläche des Absorptionsmittels zwischen 30 und 80 m²/g liegt.

5. Verfahren nach einem der Ansprüche 1 bis 4, dadurch gekennzeichnet, daß die Charge olefinischer flüssiger Kohlenwasserstoffe auf das Absorptionsmittel bei einer Temperatur zwischen ungefähr 0° C und ungefähr 90° C und mit einem Druck, der ausreicht, um die Charge in der flüssigen Phase zu halten, geleitet wird.

6. Verfahren nach einem der Ansprüche 1 bis 5, dadurch gekennzeichnet, daß eine Charge olefinischer flüssiger Kohlenwasserstoffe, die mehr als 75 % Propylen enthält, hinweggeleitet wird.

7. Verfahren nach einem der Ansprüche 1 bis 6, dadurch gekennzeichnet, daß eine Charge olefinischer Kohlenwasserstoffe, die mehr als 95 % Propylen enthält, hinweggeleitet wird.

**Claims**

1. Process for purifying liquid olefinic hydrocarbon charges incorporating propylene in particular and containing from 1 to 70 ppm of carbonyl sulphide, to remove the carbonyl sulphide, characterized by passing the liquid hydrocarbon charge, at a liquid hourly spatial velocity of between 0.2 and 15, over an absorbent material based on zinc oxide and a promoter selected from alumina, silico-aluminas, calcium oxide or mixtures thereof, the amount of the promoter not exceeding 15 % by weight of the absorbent material, and the specific surface area of the absorbent material being between 20 and 100 m²/g.

2. Purification process according to Claim 1, characterised by passing the liquid olefinic hydrocarbon charge over an absorbent material constituted by 85 to 95 % weight of zinc oxide, 3 to 10 % by weight of alumina, and 0 to 5 % by weight of calcium oxide.

3. Process according to either of Claims 1 and 2, characterized by passing the liquid olefinic hydrocarbon charge over a finely divided absorbent material the particle sizes of which do not exceed 2 mm and are preferably between 0.5 and 1.5 mm.

4. Process according to any of Claims 1 to 3, characterized in that the specific surface area of the absorbent material is between 30 and 80 m²/g.

5. Process according to any of Claims 1 to 5, characterized by passing the liquid olefinic hydrocarbon charge over the absorbent material at a temperature between about 0° C and about 90° C and at a pressure sufficient to maintain the charge in the liquid phaze.

6. Process according to any of Claims 1 to 5, characterised by passing a liquid olefinic hydrocarbon charge containing more than 75 % propylene.

7. Process according to any of Claims 1 to 6, characterised by passing a liquid olefinic hydrocarbon charge containing more than 95 % propylene.